# EUROPEAN PATENT APPLICATION

(11) **EP 3 138 562 A1**
(43) Date of publication of application: **08.03.2017**
(21) Application number: 16183490.8
(22) Date of filing: 10.08.2016
(51) Int. Cl.: A61K 31/352, A61K 31/445, A61K 31/702, A61K 33/06, A61K 35/745, A23L 33/10, A23L 33/105, A23L 33/135, A23L 33/125, A23L 33/16, A23L 33/22, A61P 5/30, A61P 15/00

(54) **NUTRACEUTICAL COMPOUND AGAINST MENOPAUSAL SYMPTOMS**

(30) Priority: 01.09.2015 IT UB20153328
(71) Applicant: Akademy Pharma S.r.l., 20135 Milano (IT)
(72) Inventor: MAGLIONE, Vincenzo, I . 20900 MONZA (Monza Brianza) (IT)
(74) Representative: Lunati & Mazzoni S.r.L.

(57) **Abstract**

A nutraceutical compound against menopausal symptoms is provided, comprising: at least one probiotic substance, at least one prebiotic substance, at least one phytoestrogen substance, DNJ, magnesium.

## Description

The object of the present invention is a nutraceutical compound against menopause symptoms of the type described in the preamble of the first claim.

As is known, menopause is the physiological event that in women corresponds to the end of the menstrual cycle and of the reproductive age. Menopause ends ovarian activity: the ovaries no longer produce follicles and estrogens, the main female hormones. Menstruation stops and a series of changes occur which affect trophic, metabolic, sexual and psychological aspects. As a world average, menopause begins between 45-50 years of age. The age of onset has extended significantly, because in ancient times menopause occurred around 40-45 years of age. Menopause can be "early" (age groups <40), premature (age 40-45), spontaneous (age <46-55) and late (age >56). Menopause can be "artificial" if it is the result of surgery or chemotherapy.

Menopause and the preceding period are characterized by initial hormonal imbalances, with an increase in the follicle stimulating hormone (FSH) levels compared to the norm. FSH rises over time and the cycles become increasingly anovulatory during the last 30 months before the onset of menopause. In most women, clinical symptoms and signs of menopause may occur with immediate, delayed or late events.

Other menopausal disorders or abnormalities are subjective or associated with vascular and other diseases. The main subjective disorder is that of hot flushes. They last from 30 seconds to 10 minutes and are accompanied by sweat, chills, and reddening of the skin. In addition, frequent somatopsychic changes occur, such as vaginal dryness, insomnia and mood swings. The symptoms are of course very variable.

A more serious concern is the increased risk of cardiovascular disease, partly in the period subsequent to menopause, partly linked to ageing. This is accompanied by an increased risk of well-known lipoprotein changes, such as the drop in HDL protective cholesterol and the increase in triglyceride levels, as well as a general trend towards an increase in blood pressure.

Women who have entered menopause are also at greater risk of vaginal candidiasis and urinary tract infection. Lastly, in addition to ageing, menopause is associated with a strong tendency to osteoporosis, with increased frequency of spontaneous or microtrauma-associated fractures.

Such menopause-related abnormalities or disorders generally compensate for the drop in hormones, particularly in estrogens, characteristic of the syndrome. The hormone replacement therapy (HRT) is the most frequently indicated therapy for managing the various symptoms.

Oral or percutaneous estrogenic treatment reduces hot flushes and generally improves bone condition.

However, in recent years, long-term studies, especially in women who have been in menopause for several years, have documented a greater risk of vascular or brain diseases after HRT (A.D. Pradhan, J.A.E. Manson, J.E. Rossouw, D.S. Siscovick, C.P. Mouton et al. - Inflammatory Biomarkers, Hormone Replacement Therapy, and Incident Coronary Heart Disease: Prospective Analysis - Women's Health Initiative Observational Study. - JAMA 288(8): 980-987, 2002).

Therefore, now there is a preference for treatments that modulate estrogen receptors, such as selective estrogen receptor modulators (SERMs), synthetic products or derivatives from plant sources. Products such as the well-known Raloxifene^{®} are capable of preventing, at least partially, bone fractures and even to a certain extent the onset of invasive breast cancer.

However, a product without potential side effects, which can face the various menopausal problems, is still missing.

In this context, the technical task underlying the present invention is to devise a nutraceutical compound against the symptoms of menopause, which is capable of substantially obviating the above-mentioned drawbacks.

Within the scope of this technical task, a major object of the invention is to obtain a nutraceutical compound against the symptoms of menopause, which mitigates or eliminates the same without substantial side effects.

The technical task and the specified objects are achieved as claimed in the appended claim 1. Preferred embodiments are described in the dependent claims. The nutraceutical compound according to the invention is at least capable of relieving the symptoms of menopause. Therefore, the invention also involves a new use of the nutraceutical compound described below for the manufacture of a food product against menopause symptoms.

The food product may be in the form of various compounds or products such as one or more of the following: pills, sachets, capsules, tablets, food in general, herbal teas, infusions and the like. Preferably, it is prepared in the form of a, preferably soluble, powder. It is suitably placed in sachets to be taken twice a day. It mainly comprises, and preferably exclusively comprises:
- at least one probiotic substance,
- at least one prebiotic substance,
- at least one phytoestrogen substance,
- 1-deoxynojirimycin (DNJ)
- magnesium.

The probiotic substance preferably comprises Bifidobacterium breve B-3, more preferably it exclusively comprises Bifidobacterium breve B-3. This is a probiotic bacterium belonging to Bacteroides. It is present in amounts comprised between 100 and 500 bn CFU, and preferably between 200 and 300 bn CFU, still more preferably of approximately 2.5 bn CFU, per 100 g of substantially dry product. Instead, the recommended daily intake of the compound according to the invention estimates that between 2 and 10 bn CFU, and preferably between 3 and 7 bn CFU, still more preferably approximately 5 bn CFU, should be taken. It is isolated and obtained according to known methods and for example marketed by *Maypro Industries^{®}* under the trade name *Morinaga b-3^{®}.*

1-Deoxynojirimycin, also designated as **DNJ,** is preferably dry-extracted naturally preferably from blackberries, silkworms or white mulberries (Morus alba). More particularly, DNJ is present inside the nutraceutical component as a dry extract of Morus alba leaves, obtained according to customary drying processes known to those skilled in the art, and in particular starting from white mulberry leaves harvested during the balsamic period to ensure the maximum content of phytoactives and using ethanol and water as the extraction solvents. It is present in amounts comprised between 5 g and 15 g, and preferably between 8 g and 12 g, still more preferably of approximately 2.5 g, per 100 g of substantially dry product. Instead, the recommended daily intake of the compound according to the invention estimates that between 20 mg and 80 mg, and preferably between 40 mg and 60 mg, even more preferably approximately 50 mg, should be taken.

The prebiotic substance preferably comprises fructo-oligosaccharides, preferably it exclusively comprises the said substance.

The main prebiotics have chemical features of water-soluble fibres. These are fructans or fructo-oligosaccharides (FOS). Among these the most important are inulin and hydrolysed guar. Other prebiotics, with a more selective use, and also features of non-caloric sweetening substances, are xylitol, sorbitol, lactulose, with strong cathartic activities too, and pectins. They are all non-digestible carbohydrates that act:
- by drawing water into the colon and hydrating the intestinal contents, thereby improving the functions of the intestine;
- by regulating the metabolic transformations of sugars and fats introduced with the diet, particularly by increasing the levels of short chain fatty acids (SCFA), which are poorly metabolised and induce the catabolism of polysaccharides. In this way, an environment is created which is suitable for the growth of anaerobic bacteria that are highly capable of catabolizing sugars.

In particular, the probiotic substance has the following characteristics:
- moisture <3.3/100 g
- dry matter >96.7 g / 100g consisting of:
- Fructo-oligosaccharides with the following composition:
   - GF2 (1-kestose) approximately 37 g / 100 fructo-oligosaccharides
   - GF3 (nystose) approximately 53 g / 100g
   - GF4 (fructofuranosyl nystose) approximately 10 g / 100g
   - Sugar <7g / 100 dry matter
- Ashes: conductometry < 0.05 g / 100g dry matter,
- pH (20°C, 30%w / v) 6.5 +1

The fructo-oligosaccharides are present in quantities by weight comprised between 8 and 10 times the Morus alba dry extract. Preferably, they are present in amounts comprised between 70 g and 100 g, and preferably between 85 g and 95 g, still more preferably of approximately 91.675 g, per 100 g of substantially dry product. Instead, the recommended daily intake of the compound according to the invention estimates that between 45 mg and 65 mg, and preferably between 52 mg and 60 mg, even more preferably approximately 56.250 mg, should be taken. Fructo-oligosaccharides (FOS) are complex carbohydrates, i.e. natural sugars in the form of starches, which are found in small amounts in various plants.

The phytoestrogen substances preferably comprise, more preferably they exclusively comprise, phytoestrogen substances obtained from soy isoflavones and / or phytoestrogen substances obtained from Angelica archa L.

Soy isoflavones are components of soy seeds, in which they are produced in significant quantities. They are present in very small quantities in other leguminous plants. They are extracted by known methods, for example, phytoestrogens are contained both in food plants (such as soy, peas and beans) and in non-food plants (such as clover).

The chemical structure of the phytoestrogens obtained therefrom is very similar to that of 17-β-estradiol. They are obtained in particular by extractive methods, and by cleaning and filtering soybean grains. These methods allow for obtaining three different products with a different protein content which can be used for the manufacture of various food products:
a) "soy flour", with 50% protein, derives directly from soybean grains after being deprived of the "fibrous coating" and of the fat component;
b) soy protein concentrate, with 70% protein content, derives from soy flour after elimination of the carbohydrate component;
c) soy protein isolate, with 90% protein content, is prepared from soybean grains after elimination of the coating and of the lipid component through aqueous extraction, and is also particularly rich in phytoestrogens.

Estradiol binds the α- and β-type estrogen receptors, ERs. The reduced production of estrogens leads to an increase in FSH, which is also believed to play a significant role in the occurrence of hot flushes. The role of phytoestrogens is based on their chemical similarity with estrogen and their ability to act as agonists and antagonists on ERs. In particular, unlike natural estrogens, phytoestrogens have higher affinity binding to ER β. This involves a series of activating phenomena in the periphery, particularly linked to a lower angiogenesis and to anti-inflammatory and antioxidant activities.

Soy isoflavones are present in quantities by weight comprised between 0.1 and 1 times the Morus alba dry extract. Preferably, they are present in amounts comprised between 2 g and 6 g, and preferably between 3 g and 5 g, still more preferably of approximately 4 g, per 100 g of substantially dry product. Instead, the recommended daily intake of the compound according to the invention estimates that between 60 mg and 100 mg, and preferably between 75 mg and 85 mg, even more preferably approximately 80 mg, should be taken.

Angelica archa L is a medicinal plant of the Umbelliferae family. It can reach two metres in height with a usually reddish thick stem and a tapering root brown on the outside and white inside. The Angelica has brownish fruits that have two seeds and three clearly visible dorsal costae. The smell of the Angelica is characteristic: very aromatic and pungent, this justifies its use in confectionery and in the liqueur industry. The Angelica contains coumarin derivatives, tannin, resins, aromatic acids, and bitter and sugary substances. Angelica infusion has a tranquillizing effect on menstrual pains and headaches and may be an excellent pack for tired eyes. It is obtained by known methods, for example by infusion in boiling water and then filtration.

With the name Erika Sinensis, in ancient times the traditional Chinese medicine indicated it for the treatment of anaemia, chronic bronchitis, asthma, rheumatism and cardiovascular disease. The Chinese or Japanese Angelica contains phytoestrogens.

Angelica archa L is in the form of a dry extract, preferably obtained by extraction from the root through infusion / filtration, and in quantities by weight comprised between 0.1 and 0.5 times the Morus alba dry extract. Preferably, they are present in amounts comprised between 1 g and 5 g, and preferably between 2 g and 3 g, still more preferably of approximately 2.5 g, per 100 g of substantially dry product. Instead, the recommended daily intake of the compound according to the invention estimates that between 40 mg and 60 mg, and preferably between 45 mg and 55 mg, even more preferably approximately 50 mg, should be taken.

Magnesium (Mg⁺⁺) is per se known and present in amounts comprised between 1 g and 5 g, and preferably between 2 g and 3 g, still more preferably of approximately 2.5 g, per 100 g of substantially dry product. Instead, the recommended daily intake of the compound according to the invention estimates that between 40 mg and 60 mg, and preferably between 45 mg and 55 mg, even more preferably approximately 50 mg, should be taken.

The iteration and the functioning of the previously described elements are as follows.

The probiotic substance, in particular Bifidobacterium breve B-3, is relatively absent from the intestinal microbiome of obese patients or from animal models with strong tendency to obesity. In particular, the probiotic substance is decreased in mice after a high-fat diet, in which there is a drop in the probiotic substance as well as in other enterobacteria. The administration thereof together with the administration of the prebiotic substance to these animals reduces inflammatory responses (rise in lipopolysaccharide) and can promote weight loss. Investigations carried out on several animal models have shown that supplementation with the probiotic substance reduces the accumulation of body and epididymal fat, also promoting a fall in the lipidemic parameters with an effect that may be considered as anti-metabolic syndrome.

The administration of the probiotic substance in humans may have the significant advantage that this bacterial strain, administered chronically, tends to modify the intestinal microbiome thus making the recipient subject less sensitive to the effects of a fat diet and with a greater tendency to reduce inflammation of the adipose tissue and consequently accumulation of fat cells. The association with the short-chain prebiotic substance is potentially very effective in increasing the activity of the probiotic substance.

The prebiotic substance is also present in the daily diet, and capable of modulating bacterial growth in the intestine. Its molecules are capable of promoting the growth of one or more bacterial strains, in particular Bifidobacteria and Lactobacilli, which have potential beneficial effects on the organism. In particular, it creates a suitable environment for the growth of the probiotic substance.

In particular, the association of said prebiotics and probiotics results in characteristics of "symbiotic foods" that have a beneficial synergistic activity on the intestinal ecoflora. The major activity of symbiotics is at the metabolic level, improving the metabolic activity of the intestinal flora on the main energy-giving elements of the diet (carbohydrates, lipids), regulating intestinal motility and, in special cases, countering the action of any pathogenic organisms.

DNJ is able to regulate the absorption of carbohydrates, resulting in reduced systemic absorption of substances capable of lowering the energy content of foods. In particular, DNJ allows the transformation of sugars (polysaccharides) into glucose to be reduced significantly and, consequently, the absorption of sugar in the blood to be slowed down. Through this mechanism, DNJ is able to reduce the weight in animal models and in humans, in particular at the dosage delivered, it promotes the reduction of glucose absorption, thereby counteracting the increase in weight, and improving the intestinal flora for a better and greater absorption of phytoestrogen substances.

Soy isoflavones involve biochemical and angiogenic effects and can lead to a decrease in the hot flushes and night sweats. The main soy isoflavones are genistein that has the highest affinity for ER β and is also active in the brain, followed by daidzein also with greater affinity for ER β, and others such as coumestrol, formononetin, and glycitein in lower concentrations.

The use of soy isoflavones is therefore particularly effective in conditions such as:
- menopausal hot flushes, where genistein increases the basal levels of NO, thereby reducing the tendency to periodic vasodilation with consequent flushes;
- increased lipoprotein oxidation, which is antagonised with a consequent potential antiartherosclerotic effect.

Angelica, in addition to the estrogenic / antiestrogenic activity described for phytoestrogens, also exerts a tonic effect on the uterus, initially causing increase in the uterine contractions and subsequently muscle relaxation. Through this mechanism, the product can exert a major preventive activity against the side effects of menopause. In addition, the product has an important spasmolytic activity that may be of interest for use in women who exhibit conditions associated with uterine flushing, in particular visceral contractions. It also has no interactions with the phytoestrogen substances, DNJ, or other components of the nutraceutical compound.

Magnesium is able to restore the electrolytic environment consequent to flushing and sweating, and is an important regulator of vascular motility. Magnesium has a significant effect on blood pressure control linked to calcium channel (Ca⁺⁺) blockade with increase of PGE1 and of NO synthesis. Clinical studies tested doses of between 190 and 973 mg / day (average dose 410 mg with follow up from 3 to 24 weeks) reporting a maximum pressure reduction of 5.6 ± 2.2 / 2.8 ± 1.9 mmHg with high variability. More recent randomized clinical trials have concluded that treatment with Mg is associated with a decrease in systolic blood pressure (SBP) of 3-4±2 mm Hg and in DBP of 2.5±1 mm Hg; the best responses are obtained in crossover studies and with daily intakes > 370 mg. The presence of magnesium (Mg⁺⁺) is beneficial in conditions such as menopausal hot flashes, where considerable sweating occurs with loss of electrolytes, and loss of magnesium is quite dreaded for the consequences on blood pressure. Experiments were carried out on rats to test the effects of the nutraceutical compound according to the invention against the metabolic syndrome and in preventing fattening.

Groups of 8 male C57BL / 6J mice of six weeks of age were administered:
- high-fat diet (44.9% fat, high fat, HF) with 35.1 % carbohydrate and 20% protein. This diet was associated with 10% skimmed milk (HF control group).
- HF diet mixed with a daily dose of 20 mg nutraceutical (low dose)
- HF diet mixed with a daily dose of 50 mg nutraceutical (high dose).

The diets were administered for a period of 8 weeks, at the end of which the body weight, the main metabolic parameters and the weight of the epididymal fat were recorded.

**Table 1. Body weight in grams (n= 8, X+SD) during the 8 weeks of treatment**

| | cholesterol | triglycerides | glucose | insulin | epididymal fat |
|---|---|---|---|---|---|
| HF controls | 164.2±17.1 | 88.3±9.6 | 195.1±17.3 | 77.4±15.6 | 2.91±1.2 |
| HF+low dose | 141.4±11.9 | 79.7±11.3 | 184±20.1 | 53.5±18.8* | 2.44±1.1° |
| HF+high dose | 126.2±8.9* | 76.1±12.3 | 176±18.6** | 41.3±13.2 **° | 2.03±1.6**° |

| | | | | | |
|---|---|---|---|---|---|
| *p <0.05 vs controls °p <0.05 vs low dose of nutraceutical **p <0.01 vs controls p <0.01 vs low dose of nutraceutical | | | | | |

**Table 2. Final values of cholesterol and triglycerides (mg / dl), glucose (mg / dl) and insulin (µU/ ml) and weight of the epididymal fat (g) in the three experimental groups at the end of the 8 weeks of treatment (n = 8, X + SD).**

| | cholesterol | triglycerides | glucose | insulin | epididymal fat |
|---|---|---|---|---|---|
| HF controls | 164.2 ±17.1 | 88.3±9.6 | 195.1±17.3 | 77.4±15.6 | 2.91±1.2 |
| HF+low dose | 141.4±11.9 | 79.7±11.3 | 184±20.1 | 53.5±18.8 * | 2.44±1.1° |
| HF+high dose | 126.2±8.9* | 76.1±12.3 | 176±18.6** | 41.3±13.2 **° | 2.03±1.6**° |

| | | | | | |
|---|---|---|---|---|---|
| *p < 0.05 vs control **p < 0.01 vs control °p< 0.05 vs low dose | | | | | |

The data from the three experimental groups show a significant weight reduction, both with the low and with the high dose of nutraceutical (Table 1). Furthermore, it can be confirmed that the weight loss is primarily in the main adipose tissue that can be modified in the course of the diet, the epididymal fat (Table 2). The assessment of the lipidemic parameters shows that both the doses of nutraceutical result in a significant decrease of cholesterol, not of triglycerides, while a marked fall was observed both for glycemia and for insulinemia. It is therefore confirmed that the experimental model is characterized by a rise in body weight and by a strong diabetogenic tendency, corrected by the nutraceutical treatment.

Other experiments were carried out to check the effect of the nutraceutical compound on hot flashes in an animal model.

For the experiments, the ovariectomized rat model was used wherein the temperature rises, especially at night, in the tail skin were examined as suggested by "E.E. Opas, S.J. Rutledge, R.L. Vogel, G. A. Rodan, A. Schmidt - Rat tail skin temperature regulation by estrogen, phytoestrogens and tamoxifen. - Maturitas 48: 463-471, 2004".

Ovariectomy or a sham surgery (leaving the ovaries intact) was performed on Sprague-Dawley rats, 250-330 g, followed by two weeks for recovery. The temperature of the normal tail skin of the sham-operated female rats was considered as the normal temperature. After stabilization of the animals for two weeks, they received a control diet with skimmed milk (control) supplemented with progressive daily doses of the nutraceutical (20 and 50 mg) for two weeks. Measurement of the skin temperature was carried out for 5 minutes on each animal kept in a flat-bottom mini-cage that allows for a minimum mobility. An SST-1 sensor (Physitemp Instruments, Inc. USA) was placed on the tail's dorsal surface approximately 1 cm from the base of the tail. The temperature measurements were carried out by using Thermalert (Th5, Physitemp Instruments, Inc. USA). All measurements were carried out during periods of darkness (at night from 22:00 to 4:00).

**Table 3: Temperature (T°C) of the tail of ovariectomized* or sham-operated° rats, treated or not with the nutraceutical at the two doses. T°C examinations over the course of the week and at times 0 (h 22), 2 (h 24), 4 (h 2 am) and 6 (h 4 am) after 2 weeks of treatment.**

| | Baseline | 2 weeks (h 18:00) | 0 (h 22) | 2 (h 24) | 4 (h 2) | 6 (h 4) |
|---|---|---|---|---|---|---|
| Sham-operated | 28.3±0.2 | 27.9±0.3 | 28.2±0.4 | 27.9±0.4 | 28.3±0.5 | 28.4±0.4 |
| Ovariecto mized | 27.9±0.2 | 29.4±0.6** | 29.5±0.5 ** | 30.1 **±0.6 | 30.3±0.5 | 30.6±0.5 ** |
| Low dose | 28.2±0.4 | 28.8±0.2* | 28.9±0.4 *° | 29.1±0.3*° | 29.2±0.2*° | 29.5±0.4 |
| High dose | 28.0±0.3 | 28.5±0.3*° | 28.2±0.3 | 28.4±0.5**° | 28.4±0.3*° | 28.4±0.4 **° |

| | | | | | | |
|---|---|---|---|---|---|---|
| *2 weeks before treatment h= hours after 22:00 (H 0) at the end of the 2 weeks *p<0.05, **p<0.01 -°p <0.05 vs low dose - °°p < 0.01 vs low dose | | | | | | |

As can be seen in Table 3, the untreated animals showed a progressive temperature rise in the 6 hours of night monitoring after the placebo diet or with the active product. The administration of the nutraceutical product resulted in a decrease of approximately 20% of the body temperature, which was significantly greater with the highest dose.

The nutraceutical compound according to the invention achieves important advantages.

In fact, the experiments presented in the patent show that the association of two different doses of the nutraceutical, transferable to the clinical condition, exhibited:
- a significant anti-obesity effect and an improvement on the blood lipid level, particularly by reducing both the cholesterolemia and the insulinemia and glycemia, thus contributing to a potential therapeutic effect on the so-called "metabolic syndrome".
- a significant effect on the experimental model of hypoestrogenemia-induced flushing, subsequent to ovariectomy in the rat. The effect occurs after direct intake of the product and persists for several hours and therefore contributes to improving the clinical picture. This effect meets the need to control hot flushes in post-menopausal women, which symptoms are very poorly tolerated.

It follows that the nutraceutical compound according to the invention is capable of greatly relieving or eliminating menopausal symptoms.

The nutraceutical compound according to the invention is also composed of natural elements and does not involve substantial side effects.

The invention is susceptible of variations falling within the scope of the inventive concept as defined by the claims.

## Claims

1. Nutraceutical compound against menopausal symptoms **characterised in that** it comprises:
- at least one probiotic substance,
- at least one prebiotic substance,
- at least one phytoestrogen substance,
- 1-deoxynojirimycin (DNJ)
- magnesium.

2. Nutraceutical compound according to claim 1, wherein said at least one probiotic substance comprises Bifidobacterium breve B-3.

3. Nutraceutical compound according to at least one previous claim, wherein said DNJ is obtained from Morus alba.

4. Nutraceutical compound according to at least one previous claim, wherein said prebiotic substance comprises fructo-oligosaccharides.

5. Nutraceutical compound according to the previous claim, wherein said fructo-oligosaccharides are present in quantities by weight between 8 and 10 times said Morus alba dry extract.

6. Nutraceutical compound according to at least one previous claim, wherein one of said phytoestrogen substances is obtained from soy isoflavones.

7. Nutraceutical compound according to the previous claim, wherein said soy isoflavones are present in quantities between 0.1 and 1 times said Morus alba dry extract.

8. Nutraceutical compound according to at least one previous claim, wherein one of said phytoestrogen substances is obtained from Angelica arch L.

9. Nutraceutical compound according to the previous claim, wherein said Angelica arch L is present in quantities between 0.1 and 0.5 times said Morus alba dry extract.

10. Use of a nutraceutical compound according to at least one previous claim for a food preparation against menopausal symptoms.
